# DEMANDE DE BREVET EUROPEEN

(11) **EP 0 545 830 A1**
(43) Date de publication de la demande: **09.06.1993**
(21) Numéro de dépôt: 92420421.7
(22) Date de dépôt: 17.11.1992
(51) Int. Cl.: A61F 2/08, A61B 17/064

(54) **Implant intra-cortical, notamment pour la fixation de ligament**

(30) Priorité: 03.12.1991 FR 9115194
(71) Demandeur: Mai, Christian, F-69006 Lyon (FR)
(72) Inventeur: Mai, Christian, F-69006 Lyon (FR)
(74) Mandataire: Laurent, Michel

(57) **Abrégé**

Cet implant intra-cortical (3), notamment par la fixation de ligaments sur des tissus osseux, est réalisé en un alliage martensitique thermo-élastique, dont les températures de transformation sont :
- température de transformation martensitique Mₛ inférieure à 5°C,
- température de transformation austénitique Aₛ supérieure à 15 °C,

Il est éduqué pour se présenter sous la forme d'une épingle en U, dont les deux branches (5) sont parallèles à une température inférieure à la température de transformation martensitique du matériau qui le compose, lesdites branches s'écartant de leur position primitive à une température supérieure à la température de transformation austénitique dudit matériau, de telle sorte à venir coopérer avec la face interne de l'os cortical du tissus osseux.

## Description

L'invention concerne un implant intra-cortical, plus particulièrement destiné à la fixation des ligaments, et ce notamment par suture.

De manière connue, un implant est une infra-structure, généralement métallique, destiné à être introduite dans un tissu par exemple osseux, soit pour renforcer un élément, soit au contraire pour le maintenir en place. Un implant intra-cortical est un implant du type en question, destiné à être inséré dans un tissu osseux en deçà de l'os cortical, et donc en partie dans l'os spongieux.

On connaît à ce jour différents modes de fixation des ligaments. Le principal mode est réalisé au moyen d'agrafes, notamment munies de picots, mais également au moyen de vis simples ou encore avec des vis à expension.

Si certes, ces modes de fixation de ligaments s'avèrent relativement simples à mettre en oeuvre, en revanche ils présentent plusieurs inconvénients majeurs. En effet, la tenue mécanique dans le temps des agrafes ou des vis repose sur l'accrochage des branches de l'agrafe ou du filetage de la vis dans l'os spongieux. Or, cette tenue mécanique s'amoindrit au cours du temps, notamment à cause du phénomène de lyse de l'os spongieux, induisant donc des risques de décrochage de l'agrafe ou de dévissage de la vis. En outre, pour les petits ligaments, ces modes de fixation sont peu adaptés. En effet, leur efficacité est réduite compte tenu des risques de rupture des ligaments qu'ils engendrent. En tout état de cause, aucun des modes et autres dispositifs de fixation connus à ce jour permet une réparation d'un ligament naturel par suture de celui-ci sur la partie osseuse.

L'objet de l'invention est de pallier ces différents inconvénients. Elle propose un implant, destiné à résoudre tout d'abord le problème du décrochage, en d'autres termes de conserver dans le temps la tenue mécanique de l'ensemble. En outre, elle vise un implant également adapté à la fixation des petits ligaments, sans risquer d'altérer ce dernier.

Cet implant intra-cortical, notamment pour la fixation de ligaments sur des tissus osseux, réalisé en un alliage martensitique thermo-élastique, dont les températures de transformation sont :
- température de transformation martensitique Mₛ inférieure à 5°C,
- température de transformation austénitique Aₛ supérieure à 15 °C,
se caractérise en ce qu'il est éduqué pour se présenter sous la forme d'une épingle en U, dont les deux branches sont parallèles à une température inférieure de la température de transformation martensitique du matériau qui les compose, lesdites branches s'écartant de leur position primitive à une température supérieure à la température de transformation austénitique dudit matériau, de telle sorte à venir coopérer avec la face interne de l'os cortical du tisseux osseux.

En d'autres termes, l'invention consiste, en utilisant les propriétés de transformation martensitique d'un alliage connu, à faire coopérer un implant cortical avec l'os cortical sur lequel il est implanté. De fait, comme on le verra dans la description qui suit, l'implant peut être entièrement noyé à l'intérieur de l'os spongieux, la tenue mécanique de l'ensemble étant obtenue par un effet de compression engendré par l'implant sur la face interne de l'os cortical au contact de l'os spongieux, et non plus directement sur l'os spongieux. Or, comme on le sait l'os cortical est rigide et stable dans le temps, et ne subit pas de lyse, sauf accident pathologique. En outre, le fait d'immerger l'implant dans l'os présente l'avantage supplémentaire d'annuler tout contact direct de l'implant avec les tissus mous environant l'os concerné.

On connaissait, par exemple dans US-A-4 485 816 et DE-A-2 703 529, des agrafes chirurgicales en forme de U à effet mémoire, dont les branches sont susceptibles de se rapprocher sous l'effet de la température. Néanmoins, ce type d'agrafes est d'application limitée aux tissus mous, et ne peut en aucun cas être utilisé aux sutures ligamentaires sur tissus osseux, voire aux sutures osseuses, compte tenu des phénomènes de lyse déjà mentionnés intervenant au niveau de l'os spongieux. En outre, ne générant aucun effet de compression au niveau de l'os cortical, elles ne peuvent induire un quelconque effet de traction au niveau de la zone de fixation ligamentaire. On ne pouvait de la sorte appliquer les enseignements de ces documents à la résolution du problème qu'entend solutionner l'invention.

Selon une forme de réalisation de l'invention, à une température supérieure à la température de transformation austénitique du matériau qui les compose, les branches s'écartent l'une de l'autre et s'alignent avec la portion de raccordement, jusqu'à constituer une tige quasiment rectiligne, qui vient se positionner parallèlement à l'os cortical.

Selon une autre forme de réalisation, à une température supérieure à la température de transformation austénitique du matériau qui les compose, les branches s'écartent l'une de l'autre et se recourbent selon deux arcs de cercle, les extrémités de chacune des branches venant au moins au niveau d'un même plan comportant ladite portion de raccordement. De la sorte, lesdites extrémités créent un effet de compression au niveau de leur point d'application, induisant le retrait à l'intérieur de l'os de la portion de raccordement, et au niveau de laquelle en général s'effectue la fixation du ligament.

Selon une autre forme de réalisation, à une température supérieure à la température de transformation austénitique du matériau qui les compose, lesdites branches se croisent, selon deux portions courbes, leurs extrémités venant au moins au niveau d'un même plan comportant ladite portion de raccordement. Dans cette autre forme de réalisation, comme dans la forme de réalisation précédente, ces branches sont destinées à créer l'effet de compression et un point d'appui au niveau de la zone d'insertion de l'implant.

Avantageusement, l'implant peut être muni d'un ou de plusieurs fils de suture solidaires dudit implant au niveau de la portion de raccordement.

Selon une forme toute particulière de l'invention, à une température supérieure à la température de transformation austénitique du matériau qui les compose,les branches se courbent jusqu'à ce que leurs extrémités libres se rapprochent en vis à vis, pour former une boucle, par exemple ronde, ovale, etc...., notamment destinée à former amortisseur.

Enfin, selon une autre forme particulière de l'invention, l'implant est constitué par deux implants du type de ceux précédemment décrits, soudés bout à bout au niveau des extrémités de leurs branches, formant ainsi une épingle aux bouts fermés.

La manière dont l'invention peut être réalisée et les avantages qui en découlent ressortiront mieux des exemples de réalisation qui suivent, donnés à titre indicatif et non limitatif à l'appui des figures annexées.

La figure 1 est une représentation schématique d'un implant conforme à l'invention à une température inférieure à la température de transformation martensitique du matériau qui le compose, mis en place dans un os.

La figure 2 est une représentation schématique de l'implant de la figure 1, à une température supérieure à la température de transformation austénitique du matériau qui le compose, par exemple typiquement à la température du corps humain.

Les figures 3, 4 et 5 représentent d'autres formes de réalisation de l'implant à une température supérieure à la température de transformation austénitique dudit matériau.

La figure 6 est une représentation d'un implant semi intra-cortical à une température supérieure à la température de transformation austénitique dudit matériau.

La figure 7 est une représentation de l'implant muni de fils de suture conformément à l'invention.

La figure 8 est une représentation schématique d'une autre forme de réalisation de l'invention, constituée par deux implants soudés bout à bout à une température inférieure à la température de transformation martensitique du matériau.

La figure 9 est une vue similaire à celle de la figure 8, à une température supérieure à la température de transformation austénitique dudit matériau.

La figure 10 est une représentation de deux implants au niveau desquels est mis en place un ligament artificel.

Selon l'invention, l'implant intra-cortical (3) comporte deux branches (5), raccordées au niveau de l'une de leurs extrémités par une portion de raccordement (4), constituant ainsi un U. Le diamètre de ces branches est fonction de la résistance souhaitée pour le ligament, et est typiquement de un millimètre, afin notamment de faciliter l'insertion de l'implant à une température inférieure à la température de transformation martensitique du matériau à base duquel il est réalisé, notamment au niveau d'un tissu osseux.

Selon une caractéristique fondamentale de l'invention, cet implant est réalisé en un matériau martensitique thermo-élastique, répondant aux critères de bio-compatibilité requis. Typiquement, ce matériau martensitique est constitué par un alliage nickel titane, ou un alliage à base de cuivre, d'aluminium et de zinc. De tels alliages sont aujourd'hui bien connus, de sorte qu'ils ne seront pas décrits ici plus en détail.

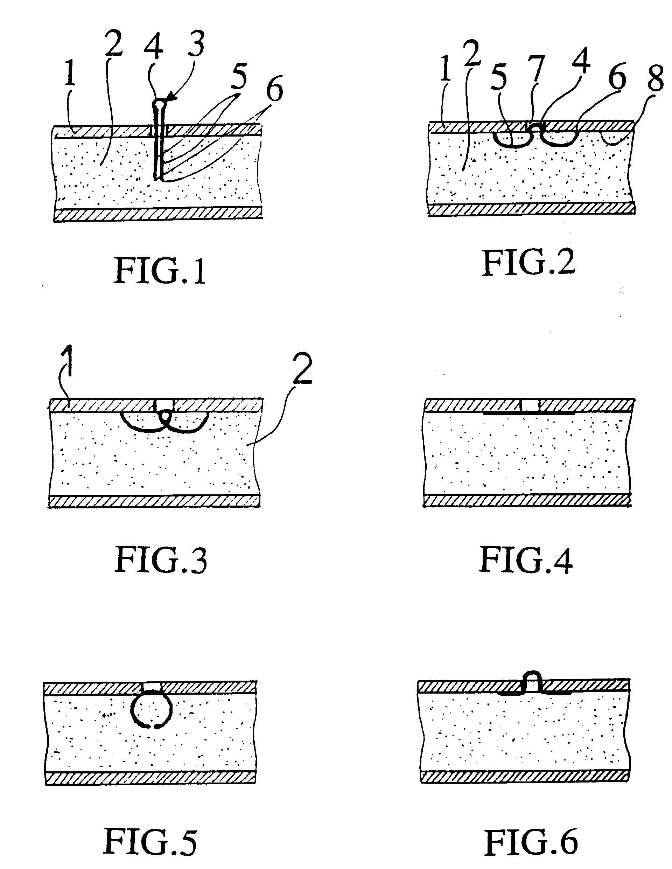

La température martensitique Mₛ du matériau est voisine de 5°C. A cette température, les branches (5) sont parallèles. Elles subissent des déformations répétées, afin d'induire un effet de mémoire de forme, qui, sera restitué lors du franchissement du seuil de température austénitique Aₛ, à savoir typiquement 25 °C. Cette mémoire de forme peut être acquise par les branches (5), en leur donnant une forme particulière à une température supérieure à la température de transformation austénitique Aₛ, puis en leur donnant une autre forme et notamment une forme droite et parallèle à une température inférieure à la température de transformation martensitique Mₛ. En répétant un certain nombre de fois ces transformations mécaniques, une mémoire de forme peut être obtenue telle qu'il sera par ailleurs décrit dans les figures 2 à 6. Par exemple dans la figure 2, la mémoire de forme des branches est courbée, lesdites branches s'écartant l'une de l'autre, et les extrémités libres (6) desdites branches venant affleurer au moins le plan contenant la portion de raccordement (4) de l'implant. Par "au moins", on entend qu'en l'absence de contrainte, lesdites extrémités (6) s'étendent au dela de la portion de raccordement (4) dudit implant. De la sorte et tel qu'on l'a représenté sur la figure 2, lesdites extrémités (6) des branches (5) exercent une compression dont le point d'application se situe sur la face interne (8) de l'os cortical (1) au niveau duquel est implanté l'implant, induisant l'insertion dans l'os spongieux (2) et en tout cas à l'intérieur de l'os de la portion de raccordement (4), et corollairement la fixation du ligament que l'implant est destiné à fixer, typiquement situé au niveau de cette portion de raccordement.

Dans une autre forme de réalisation représentée sur la figure 3, au lieu de s'écarter, les branches (5) se croisent selon également deux sections courbées, lesdites extrémités (6) des branches exerçant une nouvelle fois une compression dont le point d'application est toujours la face interne (8) de l'os cortical (1), induisant toujours l'insertion voire l'immersion de la portion de raccordement (4) dans l'os spongieux (2).

La figure 5 représente une autre forme de réalisation de l'invention, dans laquelle les deux branches (5) sont courbées, leurs extrémités libres respectives (6) venant quasiment au contact l'une de l'autre, afin de former une boucle fermée ronde, voire une autre forme fermée. Dans ce cas, l'effet de compression recherché est moins important. En revanche, la forme d'anneau au niveau des deux extrémités libres (6) joue un rôle d'amortisseur pour les ligaments susceptibles d'encaisser des efforts importants résultant de fracture.

Dans la figure 4, les deux branches (5) s'écartent à une température supérieure à la température de transformation austénitique du matériau qui les compose, jusqu'à former une tige quasiment linéaire avec la portion de raccordement (4). Dans ce cas, il n'y a pas d'effet de compression, mais la surface d'appui est très importante, et est utilisée pour la fixation de gros ligaments artificiels.

Dans la figure 6, on a représenté un implant semi-intra cortical, dans lequel on désire que la portion de raccordement (4) émerge hors de l'os cortical, et notamment hors de l'orifice (7) ménagé pour introduire l'implant lorsque celui-ci est à température inférieure à la température de transformation martensitique.

Dans tous les cas, la mise en place de l'implant suppose préalablement la réalisation d'un orifice (7) à travers l'os cortical (1). Ledit implant étant à une température inférieure à la température de transformation martensitique, il se présente sous la forme d'une épingle, telle que représentée sur la figure 1, c'est à dire les branches (5) étant parallèles pour justement permettre son insertion dans cet orifice. Progressivement, compte tenu de la température du corps humain, supérieure à la température de tranformation austénitique Aₛ du matériau qui le compose, prend la mémoire de forme selon laquelle il a été éduqué, et représentée dans l'une des figures 2 à 6. De la sorte, l'implant étant noyé ou quasiment noyé dans l'os, il n'est jamais au contact des tissus environnants et notamment de la masse musculaire.

En outre, le point de compression et d'appui s'exerçant au niveau de l'os cortical dur (1) et non plus au niveau de l'os spongieux (2), on n'observe pas de lyse de cet os cortical, de sorte que la tenue mécanique de l'implant reste constante dans le temps.

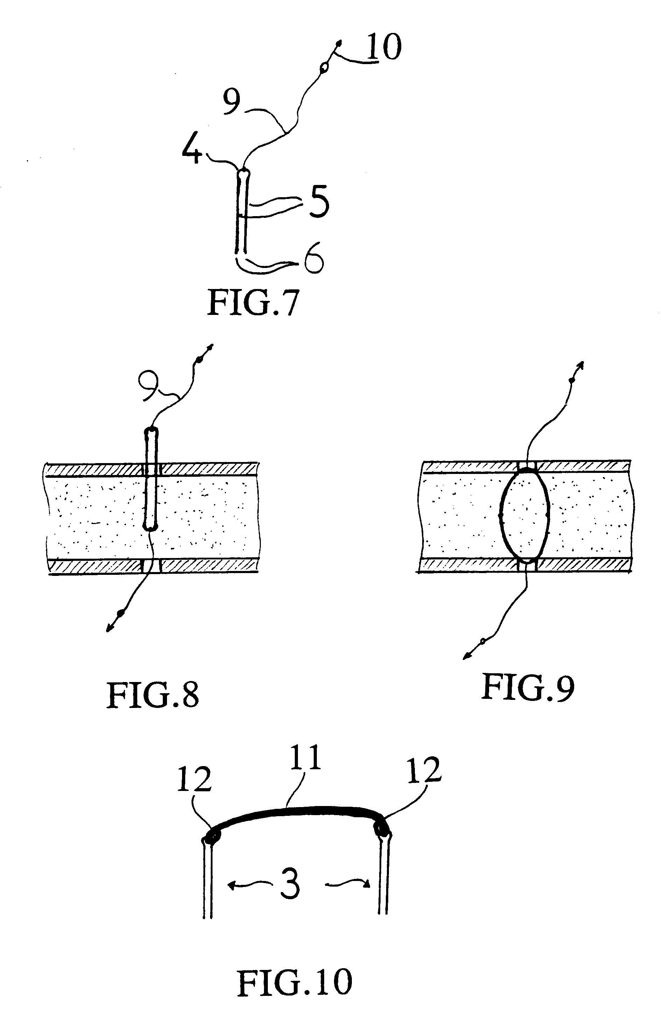

Dans une application particulière des ligaments ci-dessus décrits, on soude bout à bout les extrémités libres (6) de deux ligaments, de telle sorte qu'ils se présentent à une température inférieure à la température de transformation martensitique selon la figure 8, c'est à dire sous la forme d'une épingle fermée, dont les deux branches (5) sont parallèles. Chacune des portions de raccordement (4) est avantageusement muni d'un fil de suture (9), destiné à permettre la fixation des ligaments. La mise en place et l'utilisation d'un tel implant suppose la réalisation préalable de deux orifices (7), sensiblement diamétralement opposés au niveau de l'os cortical sur lequel doit être fixé le ou les ligaments. Sous une température supérieure à la température de transformation austénitique du matériau dudit implant, les branches (5) sont éduquées de telle sorte qu'elles se courbent, tel qu'on la représenté sur la figure 9. Cette forme particulière permet la fixation de deux ligaments au moyen d'un même implant, la courbure prise par les branches (5) s'opposant au retrait de l'implant d'un coté comme de l'autre de l'os.

Dans une forme de réalisation particulière représentée sur la figure 7, l'implant (3) est muni d'un fil de suture (9), solidaire dudit implant au niveau de la portion de raccordement (4). On a représenté par (10) l'aiguille permettant la suture dudit fil de suture (9) au niveau dudit ligament que l'on désire ainsi fixer.

L'implant conforme à l'invention peut également être utilisé pour la mise en place de ligament artificiel, notamment un ligament synthétique, bio-compatible. Il suffit pour ce faire, d'introduire un implant au niveau de chacune des deux boucles extrêmes (12) du ligament artificiel, et d'introduire lesdits implants à une température de transformation inférieure à la température de transformation martensitique Mₛ du matériau dans les orifices prévus à cet effet au niveau de l'articulation considérée (voir figure 10).

Il ressort de l'invention tout d'abord une mise en place très simple de cet implant, associé à une tenue mécanique dans le temps de grande qualité, ne pouvant s'altérer par des processus physiologiques ou biologiques. En outre, eu égard à son caractère bio-compatible, et compte tenu de son quasi-escamotage intérieur de l'os, il ne se crée pas après une mise en place de l'implant, de phénomènes d'irritation locale, inhérents au contact dudit implant avec les tissus environnants. Un tel implant s'avère donc particulièrement avantageux pour la suture de ligaments, que ceux-ci soient naturels ou synthétiques, et ce quelle que soit leur taille.

## Revendications

**1/** Implant intra-cortical (3), notamment pour la fixation de ligaments sur des tissus osseux, réalisé en un alliage martensitique thermo-élastique, dont les températures de transformation sont :
- température de transformation martensitique Mₛ inférieure à 5°C,
- température de transformation austénitique Aₛ supérieure à 15 °C,
**caractérisé** en ce qu'il est éduqué pour se présenter sous la forme d'une épingle en U, dont les deux branches (5) sont parallèles à une température inférieure à la température de transformation martensitique du matériau qui le compose, lesdites branches s'écartant de leur position primitive à une température supérieure à la température de transformation austénitique dudit matériau, de telle sorte à venir coopérer avec la face interne de l'os cortical du tissus osseux.

**2/** Implant intra-cortical selon la revendication 1, **caractérisé** en ce qu'à une température supérieure à la température de transformation austénitique du matériau qui le compose, les branches (5) s'écartent l'une de l'autre et s'alignent avec la portion de raccordement (4) du U, jusqu'à constituer une tige quasiment rectiligne.

**3/** Implant intra-cortical selon la revendication 1, **caractérisé** en ce qu'à une température supérieure à la température de transformation austénitique du matériau qui le compose, les branches (5) s'écartent l'une de l'autre et se recourbent selon deux arcs de cercle, les extrémités libres (6) de chacune des branches venant au moins au niveau d'un même plan comportant ladite portion de raccordement (4).

**4/** Implant intra-cortical selon la revendication 1, **caractérisé** en ce qu'à une température supérieure à la température de transformation austénitique du matériau qui le compose, les branches (5) se croisent, selon deux portions courbes, leurs extrémités libres (6) venant au moins au niveau d'un même plan comportant ladite portion de raccordement (4).

**5/** Implant intra-cortical selon la revendication 1, **caractérisé** en ce qu'à une température supérieure à la température de transformation austénitique du matériau qui le compose, les branches (5) se courbent, leurs extrémités libres (6) se rapprochant en vis à vis pour constituer un anneau amortisseur.

**6/** Implant intra-cortical selon la revendication 1, **caractérisé** en ce qu'à une température supérieure à la température de transformation austénitique du matériau qui le compose, la portion de raccordement (4) émerge hors de l'os (1,2) au niveau duquel il est implanté, les branches (5) s'écartant l'une de l'autre pour venir s'appuyer sur la face interne de l'os cortical (1) dudit os.

**7/** Implant intra-cortical selon l'une des revendications 1 à 6, **caractérisé** en ce qu'il est muni d'un fil (9) de suture solidaire dudit implant au niveau de la portion de raccordement (4).

**8/** Implant intra-cortical (3) notamment pour la fixation de ligaments, **caractérisé** en ce qu'il est constitué de deux ligaments (3) selon la revendication 1, soudés bout à bout au niveau de leurs extrémités libres (6), chacune des portions de racordement (4) étant munie d'un fil de suture (9), les branches (5) étant éduquées pour se courber à une température supérieure à la température de transformation austénitique du métariau qui le compose.
